# EUROPÄISCHE PATENTSCHRIFT

(11) **EP 4 495 092 B1**
(45) Veröffentlichungstag und Bekanntmachung des Hinweises auf die Patenterteilung: **10.12.2025**
(21) Anmeldenummer: 23186740.9
(22) Anmeldetag: 20.07.2023
(51) Int. Cl.: C07C 2/06, C07C 11/02, C07C 45/50, C07C 47/02, C07C 45/82

(54) **VERFAHREN ZUR HYDROFORMYLIERUNG VON DI-ISOBUTEN UND EINEM C4- BIS C7-OLEFIN**
PROCESS FOR THE HYDROFORMYLATION OF DI-ISOBUTENE AND A C4-C7 OLEFIN
PROCÉDÉ D'HYDROFORMYLATION DE DI-ISOBUTÈNE ET D'UNE OLÉFINE C4- À C7

(43) Veröffentlichungstag der Anmeldung: 22.01.2025
(73) Patentinhaber: Evonik Oxeno GmbH & Co. KG, 45772 Marl (DE)
(72) Erfinder: KUCMIERCZYK, Peter, 44628 Herne (DE); TERMÜHLEN, Maren, 44135 Dortmund (DE); REINHARDT, Annika, 45770 Marl (DE); RIX, Armin Matthias, 45770 Marl (DE); FRANKE, Robert, 45772 Marl (DE); FRIDAG, Dirk, 45721 Haltern am See (DE); SALE, Anna Chiara, 45657 Recklinghausen (DE); MARKOVIC, Ana, 45721 Haltern am See (DE)
(74) Vertreter: Evonik Patent Association

(56) Entgegenhaltungen:
- WO-A1-2021/160448
- "The Hydroformylation Reaction", 1 January 2000, ISBN: 978-0-471-39568-3, article IWAO OJIMA ET AL: "The Hydroformylation Reaction", pages: 1 - 354, XP055409750, DOI: 10.1002/0471264180.or056.01

## Beschreibung

Die vorliegende Erfindung betrifft ein Verfahren zur Hydroformylierung von Di-isobuten und einem C4- bis C7-Olefin in einer gemeinsamen Reaktionszone. Die Hydroformylierung wird mit Synthesegas in Gegenwart eines homogenen Katalysatorsystems, welches zumindest Co oder Rh und optional einen phosphorhaltigen Liganden umfasst, durchgeführt.

Di-isobuten ist ein technisch relevantes Erzeugnis, welches durch Dimerisierung von Isobuten gewonnen wird. Di-isobuten besteht aus den Isomeren 2,4,4-Trimethylpent-1-en (nachfolgend auch: TMP1) und 2,4,4-Trimethylpent-2-en (nachfolgend auch: TMP2) mit einer Massenverteilung TMP1 : TMP2 im Bereich von etwa 78:22 bis 81 : 19 (Gleichgewichtsverteilung). Technische Gemische, die C4-Olefine enthalten, sind Leichtbenzinfraktionen aus Raffinerien, C4-Fraktionen aus FC- oder Steamcrackern, Gemische aus Fischer-Tropsch-Synthesen, Gemische aus Dehydrierung von Butanen und durch Metathese oder aus anderen technischen Prozessen entstandene Gemische. C5-Olefine, also Pentene, sind in Leichtbenzinfraktionen aus Raffinerien oder Crackern enthalten. Die höheren Olefine können insbesondere durch Oligomerisierungsreaktionen gewonnen werden. Sowohl Di-isobuten als auch C4- bis C7-Olefine können durch die Hydroformylierung zu Wertprodukten wie den bei der Hydroformylierung gebildeten Estern umgesetzt werden.

Das Problem bei derartigen Verfahren ist, dass eigenständige Produktionsanlagen vorhanden oder gebaut werden und dass diese Anlagen mit entsprechendem Aufwand betrieben werden müssen.

Da die Märkte für petrochemische Produkte zum Teil recht volatil sein, sind eigene Produktionsanlagen für jede der genannten Olefine kaum wirtschaftlich zu betrieben. Ein weiterer Nachteil ist, dass ein ressourcenschonender Betrieb von mehreren Produktionsanlagen kaum möglich ist, weil alle Anlagen instandgehalten werden müssen. Das ist nicht nur ein wirtschaftlicher und personeller Aufwand, sondern erfordert auch gewisse Mengen an Energie wie Strom oder Wärmeträger.

Die Aufgabe der vorliegenden Erfindung war deshalb die Bereitstellung eines Verfahrens, welches die vorgenannten Probleme nicht aufweist. Insbesondere sollten sowohl Di-isobuten als auch C4-bis C7-Olefine per Hydroformylierung ressourcenschonender zu Wertprodukten umgesetzt werden können.

Die zugrundeliegende Aufgabe konnte durch das in Anspruch 1 beschriebene Verfahren gelöst werden. Bevorzugte Ausführungsformen sind in den abhängigen Ansprüchen angegeben.

Erfindungsgemäß umfasst das Verfahren zur Hydroformylierung von Di-isobuten und einem C4- bis C7-Olefin zumindest die folgenden Schritte:
a. Bereitstellen eines Di-isobutenstroms, enthaltend 2,4,4-Trimethylpent-2-en und 2,4,4-Trimethylpent-1-en, und Bereitstellen eines Olefinstroms, enthaltend das C4- bis C7-Olefin;
b. Hydroformylierung von Di-isobuten und dem C4- bis C7-Olefin mit Synthesegas in Gegenwart eines homogenen Katalysatorsystems, welches zumindest Co oder Rh und optional einen phosphorhaltigen Liganden umfasst, in einer Reaktionszone unter Erhalt eines, vorzugsweise flüssigen Produktgemisches, welches zumindest die durch die Hydroformylierung gebildeten Aldehyde 3,5,5-Trimethylhexanal und einem C5- bis C8-Aldehyd, das homogene Katalysatorsystem und nicht umgesetzte Olefine, d. h. nicht umgesetztes Di-isobuten und nicht umgesetzte C4- bis C7-Olefine umfasst;
c. Abtrennen des homogenen Katalysatorsystems aus dem vorzugsweise flüssigen Produktgemisch unter Erhalt eines Rohproduktgemisches, welches zumindest die durch die Hydroformylierung gebildeten Aldehyde 3,5,5-Trimethylhexanal und einem C5- bis C8-Aldehyd und die nicht umgesetzten Olefine umfasst; und
d. Destillative Aufarbeitung des Rohproduktgemisches in mindestens einer Destillationskolonne zur Abtrennung der nicht umgesetzten Olefine unter Erhalt eines Aldehydgemischs, welches die gebildeten Aldehyde 3,5,5-Trimethylhexanal und einem C5- bis C8-Aldehyd enthält, wobei vorzugsweise die nicht umgesetzten Olefine abgetrennt und zur Hydroformylierung in Schritt b zurückgeführt wird.

Das erfindungsgemäße Verfahren betrifft also die gleichzeitige Umsetzung von Di-isobuten und C4- bis C7-Olefinen in einer einzigen gemeinsamen Reaktionszone. Ein solches Verfahren hat eine Vielzahl von Vorteilen.

Durch das beschriebene Verfahren ist es möglich, dass auf Märkte, insbesondere geringer Produktionsmengen, flexibel reagiert werden kann. Zudem ist nur eine Produktionsanlage notwendig, die zudem selbst bei schwankenden Marktbedürfnissen effizienter und damit ressourcenschonender betrieben werden kann. Durch die Besonderheit der Siedereihenfolge ist es außerdem möglich, die anfallenden Produkte der jeweils eingesetzten Olefine zu trennen, während die Edukte wieder direkt oder nach zusätzlicher Aufarbeitung der Reaktion zugeführt werden können.

Der in Schritt a bereitgestellte Di-isobutenstrom enthält 2,4,4-Trimethylpent-2-en und 2,4,4-Trimethylpent-1-en. In einer bevorzugten Ausführungsform beträgt der Anteil von 2,4,4-Trimethylpent-1-en im Di-isobutenstrom mindestens 60 Mol%, vorzugsweise mindestes 70 Mol% bezogen auf den gesamten Di-isobutenstrom. Derartige Ströme können Di-isobutenströme sein, die mittels Dimerisierung aus Isobuten oder Isobuten-haltigen Kohlenwasserstoffgemischen hergestellt wurden, beispielsweise nach dem in EP 1 360 160 B1 offenbarten Verfahren. Weiterhin können die hier einzusetzenden Di-isobutenströme als nicht reagierte Restströme bei Carbonylierungsverfahren anfallen, beispielsweise bei der Alkoxycarbonylierung oder bei der Hydroformylierung. WO2021/160448 A1 offenbart ein Verfahren zur Hydroformylierung von Di-isobuten.

Neben dem Di-isobutenstrom wird in Schritt a ein Olefinstrom bereitgestellt, der das im erfindungsgemäßen Verfahren eingesetzte C4- bis C7-Olefin enthält. In einer bevorzugten Ausführungsform wird in der vorliegenden Erfindung ein Olefinstrom eingesetzt, der C4-Olefine enthält, besonders bevorzugt ein C4-Olefinstrom. Entsprechende Ströme sind dem Fachmann bekannt und großtechnisch verfügbar Olefineströme, die C4-Olefine enthalten, sind beispielsweise Leichtbenzinfraktionen aus Raffinerien, C4-Fraktionen aus FC- oder Steamcrackern, Gemische aus Fischer-Tropsch-Synthesen, Gemische aus Dehydrierung von Butanen oder durch Metathese oder aus anderen technischen Prozessen entstandene Ströme. Beispielsweise können für das erfindungsgemäße Verfahren geeignete C4-Olefinströme aus der C4-Fraktion eines Steamcrackers gewonnen werden. C5-Olefine, also Pentene, sind in Leichtbenzinfraktionen aus Raffinerien oder Crackern enthalten.C6-Olefine können beispielsweise durch Dimerisierung von Propen gewonnen werden. C7-Olefine können beispielsweise durch Dimerisierung von Propylen und Buten gewonnen werden.

Die in Schritt a bereitgestellten Ströme, d. h. der Di-isobutenstrom und der C4- bis C7-Olefinstrom werden zur Hydroformylierung in Schritt b geleitet. Dabei können die Ströme einzeln und separat zur Hydoroformylierung in Schritt b geleitet werden oder vorher vermischt werden. Vorzugsweise werden der Di-isobutenstrom und der C4- bis C7-Olefinstrom vor der Hydroformylierung in Schritt b vermischt. In einer besonders bevorzugten Ausführungsform der vorliegenden Erfindung werden sogar der Di-isobutenstrom, der C4- bis C7-Olefinstrom und das homogene Katalysatorsystem vor der Hydroformylierung in Schritt b vermischt, insbesondere in einem geeigneten Mischbehälter. Sofern es einen Recyclestrom zur Reaktion gibt, also z. B. durch Rückführung des Katalysatorsystems, kann dieser Recyclestrom ebenfalls zum Mischbehälter geführt werden.

Die Di-isobutene, d. h. 2,4,4-Trimethylpent-2-en und 2,4,4-Trimethylpent-1-en, werden in Schritt b mit Synthesegas (Mischung aus Kohlenmonoxid (CO) und Wasserstoff (H₂)) zu einem Aldehyd umgesetzt. Die Anzahl der Kohlenstoffatome im Aldehyd erhöht sich dabei im Vergleich mit dem eingesetzten Di-isobuten um 1 Kohlenstoffatom. Aus den Di-isobutenen (8 Kohlenstoffatome) entsteht demnach ein Aldehyd mit 9 Kohlenstoffatomen, nämlich 3,5,5-Trimethylhexanal. Demnach entsteht aus dem C4- bis C7-Olefin ein C5- bis C8-Aldehyd durch die erfindungsgemäße Hydroformylierung.

Das Synthesegas für das erfindungsgemäße Verfahren kann in unterschiedlichen Mischungsverhältnissen von Kohlenmonoxid und Wasserstoff eingesetzt werden. Das molare Verhältnis zwischen Synthesegas und dem eingesetzten Kohlenwasserstoffstrom, der die zu hydroformylierende Olefine enthält, sollte zwischen 6:1 und 1:1, vorzugsweise zwischen 3:1 und 1:1, besonders bevorzug zwischen 2:1 und 1:1 liegen.

Die Hydroformylierung kann optional in Anwesenheit eines dem Fachmann bekannten zusätzlichen Lösemittels durchgeführt wird, vorzugsweise wird jedoch kein zusätzliches Lösemittel verwendet, sondern das eingesetzte Olefin fungiert bei der Hydroformylierung als Lösemittel.

Das bei der Hydroformylierung einsetzbare homogene Katalysatorsystem enthält Co oder Rh, vorzugsweise Rh, und optional einen phosphorhaltigen Liganden. Entsprechende Katalysatorsysteme sind dem Fachmann geläufig. Der Einsatz eines phosphorhaltigen Liganden ist bevorzugt. In einer besonders bevorzugten Ausführungsform umfasst oder besteht das homogene Katalysatorsystem aus Rh und einem phosphorhaltigen Liganden. Geeignete Liganden für die erfindungsgemäßen Katalysatorsysteme sind dem Fachmann bekannt. Der phosphorhaltige Ligand für das erfindungsgemäße Katalysatorsystem ist vorzugsweise ein Phosphin (z. B. TPP (Triphenylphosphin), ein Monophosphit (z. B. Alkanox 240 (Tris(2,4-di-tert-butylphenyl)phosphit) oder ein Bisphosphit (z. B. Biphephos). Es können auch Mischungen von Liganden eingesetzt werden.

Die Temperatur bei der homogen katalysierten Hydroformylierung liegt vorzugsweise im Bereich von 80 bis 250 °C, weiterhin bevorzugt im Bereich von 90 bis 225 °C und besonders bevorzugt im Bereich von 100 bis 210 °C. Der Druck bei der homogen katalysierten Hydroformylierung liegt vorzugsweise im Bereich von 100 bis 350 bar, weiterhin bevorzugt im Bereich von 175 bis 325 bar und besonders bevorzugt im Bereich von 200 bis 300 bar.

Der Druck bei der Hydroformylierung entspricht üblicherweise dem Gesamtgasdruck. Der Gesamtgasdruck meint im Rahmen der vorliegenden Erfindung die Summe der vorliegenden Drücke aller vorhandenen gasförmigen Stoffe, also den Druck der (gesamten) Gasphase. Im vorliegenden Verfahren entspricht dies insbesondere der Summe der Partialdrücke von CO und H₂, d. h. der Gesamtgasdruck ist dann der Synthesegasdruck.

Homogene katalysierte Hydroformylierungen können als Flüssigaustragsverfahren ("liquid recycle") oder als Gasaustragsverfahren ("gas recycle") betrieben werden. Beide Verfahrensvarianten sind dem Fachmann bekannt und in vielen Lehrbüchern beschrieben. Eine konkrete Auswahl eines solchen Verfahrens ist im Rahmen der vorliegenden Erfindung nicht notwendig, weil das Verfahren grundsätzlich auf beide Arten durchgeführt werden kann. Wichtig bei der homogenen Katalyse ist allenfalls noch die Abtrennung des Katalysatorsystems aus dem Reaktionsaustrag. Bei einem flüssigen Austrag ist dies beispielsweise über Flashverfahren oder Membrantrennung möglich. Bei einem gasförmigen Austrag beispielsweise mittels Kondensation und/oder Auswaschen. Auch dies ist dem Fachmann bekannt und darf keiner ausführlichen Erläuterung. Die weitere Aufarbeitung des Reaktionsaustrags, insbesondere die Abtrennung des Reaktionsprodukts, ist dem Fachmann ebenfalls geläufig und kann beispielsweise mittels eines thermischen Trennverfahrens wie der Destillation erfolgen. Thermische Trennung bzw. thermische Trennverfahren im Sinne der vorliegenden Erfindung meint ein Trennverfahren, bei dem die Trennung anhand des Siedepunktes erfolgt.

Die Hydroformylierung in Schritt b findet in einer geeigneten Reaktionszone statt. Die Reaktionszone für die Umsetzung umfasst mindestens einen Reaktor, kann aber auch aus zwei oder mehr Reaktoren bestehen, die parallel oder in Reihe geschaltet angeordnet sind. Der mindestens eine Reaktor kann insbesondere aus der Gruppe, bestehend aus einem Rührkesselreaktor, einem Schlaufenreaktor, einem Jet-Loop-Reaktor, einem Blasensäulenreaktor oder Kombinationen daraus, ausgewählt sein. Sind mehrere Reaktoren vorhanden können die Reaktoren gleich oder unterschiedlich sein.

Durch die beschriebene Hydroformylierung in Schritt b wird ein vorzugsweise flüssiges Produktgemisch erhalten, das zumindest den durch die Hydroformylierung gebildeten Aldehyde 3,5,5-Trimethylhexanal und das C5- bis C8-Aldehyhd, das homogene Katalysatorsystem und die nicht umgesetzten Olefine, d. h. Di-isobutene und C4- bis C7-Olefine, umfasst.

Das so erhaltene vorzugsweise flüssige Produktgemisch wird dem nachfolgenden Schritt c zugeführt, um das homogene Katalysatorsystems aus dem Produktgemisch abzutrennen. Vor dem Zuführen des Produktgemisch können bereits leichtsiedende Komponenten beispielsweise leichtsiedende Nebenprodukte abgetrennt werden, beispielsweise mittels thermischer Trennung (Flash, Destillation, o. ä.), wofür eine Entspannung des unter hohem Druck stehenden Produktgemischs notwendig sein kann.

In einer bevorzugten Ausführungsform der vorliegenden Erfindung erfolgt weiterhin eine Kühlung des Produktgemischs vor der Abtrennung in Schritt c auf eine Temperatur zwischen 40 und 100 °C, vorzugsweise zwischen 50 und 95 °C, besonders bevorzugt zwischen 60 und 90°C. Dazu wird eine geeignete Kühlvorrichtung benötigt. Das Kühlen erfolgt insbesondere mit einem Austragskühler. Als zweckmäßig haben sich beispielsweise Rohrbündelwärmetauscher erwiesen, wobei das Reaktionsgemisch vorzugsweise durch die Rohe und das Kühlmedium vorzugsweise durch den Mantel des Wärmetauschers geführt wird.

Die Kühlung des Produktgemischs sorgt für eine Verringerung des Katalysatormetall-Einsatzfaktors. Das Problem ist, dass während einer Hydroformylierung und der nachfolgenden

Abtrennung immer ein kleiner Teil des Metalls, insbesondere des Rhodiums auf verschiedene Weise verloren geht. Aufgrund der hohen Preise für die einzusetzenden Metalle, insbesondere des Metalls erhöht das die Prozesskosten, weil die Verluste durch Nachdosierung ausgeglichen werden müssen. Das erfindungsgemäße Kühlen hat nun jedoch den Effekt, dass der Einsatzfaktor sinkt, also weniger Katalysatormetall, insbesondere Rhodium verloren geht und deshalb weniger nachdosiert werden muss. Die Prozesskosten können also auf diese Weise erheblich gesenkt werden.

Die Abtrennung des homogenen Katalysatorsystems unter Erhalt des Rohproduktgemisches in Schritt c kann mithilfe verschiedener Trennverfahren erfolgen, beispielsweise mittels thermischer Trennung und/oder durch Membrantrennung. Entsprechende Verfahren sind dem Fachmann geläufig. Es ist bevorzugt, wenn zuerst eine thermische Trennung, beispielsweise eine Verdampfung und anschließend eine Membrantrennung erfolgt. Bei der Verdampfung gehen hauptsächlich Produktaldehyde (Trimethylhexanal und C5- bis C8-Aldehyd) und nicht umgesetzten Di-isobutenen und C4- bis C7-Olefine als Rohproduktgemisch über Kopf. Im Sumpf fällt eine Hochsiederphase an, die den homogenen Katalysator, Trimethylhexanal und teilweise C5- bis C8-Aldehyde und eventuell entstandene Hochsieder enthält. Die Hochsiederphase kann dann einer Membrantrennung unterzogen werden, um mögliche Hochsieder auszuschleusen. Bei einer Membrantrennung fallen bekanntermaßen ein Retentat und ein Permeat an. Das Katalysatorsystem wird sich im Retentat anreichern. Das Permeat kann einer weiteren Aufarbeitung zugeführt werden.

Das Retentat enthält dabei das homogene Katalysatorsystem. Es ist erfindungsgemäße bevorzugt, dass das Retentat zur Hydroformylierung in Schritt b bzw. zur Reaktionszone, wo die Hydroformylierung durchgeführt wird, zurückgeführt wird. So kann das Katalysatorsystem wiederverwendet werden. Bei der bevorzugt kontinuierlichen Durchführung des beanspruchten Verfahrens entsteht so ein Katalysatorkreislauf, wo wenn überhaupt nur geringfügige prozessbedinge Katalysatorverluste ausgeglichen werden müssen. Sofern der Di-isobutenstrom, das C4- bis C7-Olefin und das homogene Katalysatorsystem gemäß der bevorzugten Ausführungsform vor der Hydroformylierung in Schritt b insbesondere in einem geeigneten Mischbehälter vermischt werden wird, wird das Retentat zum Mischbehälter geführt.

Bei der Membrantrennung kann jedes geeignete Membranmaterial eingesetzt werden. Vorzugsweise wird bei der Membrantrennung nach der Verdampfung des erfindungsgemäßen Verfahrens ein OSN-Membranmaterial (OSN = Organic Solvent Nanofiltration) eingesetzt. Ein solches Membranmaterial besteht vorzugsweise zumindest aus einer trennaktiven Schicht (auch: aktiven Trennschicht) und einer Unterstruktur, worauf sich die trennaktive Schicht befindet. Bevorzugt besteht das erfindungsgemäße Membranmaterial zumindest aus einer trennaktiven Schicht und einer Unterstruktur.

Die Unterstruktur weist vorzugsweise eine poröse Struktur auf, die für das durch die trennaktive Schicht gelangte Permeat durchlässig ist. Die Unterstruktur hat eine stabilisierende Funktion und dient als Träger für die trennaktiven Schicht. Die Unterstruktur kann grundsätzlich aus jedem geeigneten porösen Material bestehen. Entsprechende Materialien sind dem Fachmann geläufig. Voraussetzung ist jedoch, dass das Material säure- und basenstabil ist. Die Unterstruktur kann auch aus dem gleichen Material bestehen wie die trennaktive Schicht. Bevorzugte Materialien für die Unterstruktur sind Kunststoffe wie Polypropylen (PP), Polyethylen (PE) oder nicht-Kondensationspolymere, die sich nicht hydrolytisch oder alkoholytisch spalten lassen, wie Polysulfone, Polytetrafluorethylen (PTFE), Polyethersulfon (PES), Polyvinylidenfluorid (PVDF) oder Polyacrylnitril (PAN).

Die erfindungsgemäße trennaktive Schicht besteht vorzugsweise aus einem PAEK-Polymer (Polyaryletherketon). PAEK zeichnet sich dadurch aus, dass innerhalb der Widerholungseinheit Arylgruppen abwechselnd über eine Etherfunktionalität und eine Ketonfunktionalität verknüpft sind. Eine erfindungsgemäß bevorzugte trennaktive Schicht besteht aus PEEK (Polyetheretherketon). Als trennaktive Schicht können insbesondere bevorzugt PEEK-Polymere mit einem Sulfonierungsgrad von weniger als 20%, besonders bevorzugt mit einem Sulfonierungsgrad von weniger als 10% eingesetzt werden. Die entsprechenden PEEK-Polymere und deren Herstellung sind in der WO 2015/110843 A1 beschrieben.

Die Membrantrennung in Schritt c wird vorzugsweise bei einer Temperatur im Bereich von 25 bis 100°C, weiterhin bevorzugt im Bereich von 30 bis 80°C und besonders bevorzugt im Bereich von 40 bis 70°C durchgeführt. Um das Produktgemisch auf die bei der Membrantrennung bevorzugte vorherrschende Temperatur zu bringen, kann das Produktgemisch gekühlt werden. Neben einer aktiven Kühlung unter Verwendung eines Kühlmediums, kann die Kühlung auch über einen Wärmetauscher erreicht werden, wo Wärmeenergie auf einen anderen Strom übertragen wird, wodurch sich das Produktgemisch abkühlt und der andere Strom erhitzt wird.

Der Transmembrandruck (TMP) liegt bei der Membrantrennung in Schritt c vorzugsweise im Bereich von 10 bis 60 bar, weiterhin bevorzugt im Bereich von 15 bis 55 bar, besonders bevorzugt im Bereich von 40 bis 50 bar. Der permeatseitige Druck kann dabei oberhalb des atmosphärischen Drucks liegen und vorzugsweise bis 15 bar, vorzugsweise 2 bis 7 bar betragen. Aus der Differenz von TMP und permeatseitigem Druck ergibt sich der retentatseitige Druck. In einer bevorzugten Ausführungsform sollte bei den Druckverhältnissen und insbesondere beim permeatseitigen Druck darauf geachtet werden, dass der Druck so eingestellt wird, dass eine Verdampfung nach dem Durchtritt durch die Membran vermieden wird. Ein Verdampfen könnte zu einer instabilen Fahrweise führen.

Im anschließenden Schritt d erfolgt die destillative Aufarbeitung des Rohproduktgemisches in mindestens einer Destillationskolonne zur Abtrennung der nicht umgesetzten Olefine (Di-isobutene und C4- bis C7-Olefin). Dabei wird ein Aldehydgemisch erhalten, welches die gebildeten Aldehyde 3,5,5-Trimethylhexanal und C5- bis C8-Aldehyd enthält.

Bei der destillativen Aufarbeitung des Rohproduktgemisches in Schritt d fallen die nicht umgesetzten Olefine, d. h. nicht umgesetzte Di-isobutene und nicht umgesetzte C4- bis C7-Olefine, am Kopf der mindestens einen Destillationskolonne an. Das Gemisch der gebildeten Aldehyde fällt folglich im Sumpf der mindestens einen Destillationskolonne an. Der Kopfstrom, der die nicht umgesetzten Olefine enthält, kann zur Hydroformylierung in Schritt b bzw. zur Reaktionszone zurückgeführt werden. Ist eine Vermischung der eingesetzten Komponenten vor der Hydroformylierung vorhanden, wird der Kopfstrom natürlich zur Vermischung geführt. Dadurch ist ein kontinuierlicher Betrieb des erfindungsgemäßen Verfahrens unter höchstmöglicher Ausbeute möglich. Aus dem zurückgeführten Kopfstrom kann ein Purge abgezogen werden, um leichtsiedende Nebenprodukte aus dem Verfahren auszuschleusen.

Die destillative Aufarbeitung zur Abtrennung der nicht umgesetzten Olefine in Schritt d kann in einer Destillationskolonne erfolgen. Denkbar wäre, dass die destillative Aufarbeitung zur Abtrennung der nicht umgesetzten Olefine in Schritt d in mehreren Destillationskolonnen erfolgt. Dies würde aber einen deutlichen höheren apparativen Aufwand bedeuten. Bevorzugt ist deshalb, dass die destillative Aufarbeitung in Schritt d in einer einzigen Destillationskolonne erfolgt.

Der Druck in der Destillationskolonne bei der destillativen Aufarbeitung in Schritt d liegt vorzugsweise im Bereich von 0,3 bis 2 bar, weiterhin bevorzugt im Bereich von 0,4 bis 1 bar, besonders bevorzugt im Bereich 0,5 bis 0,7 bar beträgt. Die Temperatur im Sumpf der Destillationskolonne bei der destillativen Aufarbeitung in Schritt d liegt vorzugsweise im Bereich von 80 °C bis 160 °C. Die Temperatur am Kopf der Destillationskolonne bei der destillativen Aufarbeitung in Schritt d liegt vorzugsweise im Bereich von 30 bis 80 °C. Weiterhin ist es bevorzugt, dass das Rücklaufverhältnis in der Destillationskolonne zwischen 1 und 2 beträgt. Die Destillationskolonne für die Abtrennung in Schritt d umfasst vorzugsweise 10 bis 30 theoretische Stufen umfasst. Die Destillationskolonne kann dabei Hochleistungsstrukturpackungen enthalten. Entsprechende Hochleistungsstrukturpackungen sind dem Fachmann bekannt.

Durch die destillative Aufarbeitung wird, wie erwähnt, ein Aldehydgemisch erhalten, das die aus dem Di-isobuten und dem C4- bis C7-Olefin gebildeten Aldehyde enthält. Um beide Aldehyde als möglichst reine Stoffe zu erhalten kann ein weiterer Destillationsschritt durchgeführt werden, um die Aldehyde voneinander zu trennen. Der Aldehyd aus dem C4- bis C7-Olefin wird dabei am Kopf der Destillationskolonne und der Aldehyd aus dem Di-isobuten am Sumpf der Destillationskolonne anfallen. Denkbar sind auch Trennwandkolonnen um an verschiedenen Stellen die Aldehyde als Reinkomponenten innerhalb einer Kolonne zu gewährleisten. Entsprechende technische Auslegung einer Trennwandkolonne sind dem Fachmann bekannt und großtechnisch verfügbar.

Das vorliegende Verfahren eignet sich insbesondere zur Hydroformylierung von Di-isobuten und C4- bis C7-Olefinen. Bestimmte Kombinationen von Olefinen sind im Rahmen der vorliegenden Erfindung besonders bevorzugt:
In einer bevorzugten Ausführungsform betrifft das Verfahren die Hydroformylierung von Di-isobuten und einem C4-Olefin, d. h. 1-Buten, cis- und/oder trans-2-Buten, Isobuten oder Mischungen davon. Der aus dem Di-isobuten gebildete Aldehyd ist 3,5,5-Trimethylhexanal. Aus dem C4-Olefin entsteht Pentanal, 2-Methylbutanal oder 3-Methylbutanal. Wird ein Gemisch von Butenen eingesetzt wird dementsprechend auch eine Mischung der genannten Aldehyde erhalten.

In einer besonders bevorzugten Ausführungsform betrifft das Verfahren die Hydroformylierung von Di-isobuten und Isobuten. Der aus dem Di-isobuten gebildete Aldehyd ist 3,5,5-Trimethylhexanal. Aus dem Isobuten entsteht 3-Methylbutanal.

## Patentansprüche

1. Verfahren zur Hydroformylierung von Di-isobuten und einem C4- bis C7-Olefin, wobei das Verfahren zumindest die folgenden Schritte umfasst:
a. Bereitstellen eines Di-isobutenstroms, enthaltend 2,4,4-Trimethylpent-2-en und 2,4,4-Trimethylpent-1-en, und Bereitstellen eines Olefinstroms, enthaltend das C4- bis C7-Olefin;
b. Hydroformylierung von Di-isobuten und dem C4- bis C7-Olefin mit Synthesegas in Gegenwart eines homogenen Katalysatorsystems, welches zumindest Co oder Rh und optional einen phosphorhaltigen Liganden umfasst, in einer Reaktionszone unter Erhalt eines vorzugsweise flüssigen Produktgemisches, welches zumindest die durch die Hydroformylierung gebildeten Aldehyde 3,5,5-Trimethylhexanal und einem C5- bis C8-Aldehyd, das homogene Katalysatorsystem und nicht umgesetzte Olefine umfasst;
c. Abtrennen des homogenen Katalysatorsystems aus dem vorzugsweise flüssigen Produktgemisch unter Erhalt eines Rohproduktgemisches, welches zumindest die durch die Hydroformylierung gebildeten Aldehyde 3,5,5-Trimethylhexanal und einem C5- bis C8-Aldehyd und die nicht umgesetzten Olefine umfasst; und
d. Destillative Aufarbeitung des Rohproduktgemisches in mindestens einer Destillationskolonne zur Abtrennung der nicht umgesetzten Olefine unter Erhalt eines Aldehydgemischs, welches die gebildeten Aldehyde 3,5,5-Trimethylhexanal und einem C5- bis C8-Aldehyd enthält.

2. Verfahren nach Anspruch 1, wobei die Hydroformylierung in Schritt b bei einer Temperatur von 90 bis 250 °C, vorzugsweise von 120 bis 200 °C, besonders bevorzugt von 120 bis 170 °C durchgeführt wird.

3. Verfahren nach Anspruch 1 oder 2, wobei die Hydroformylierung in Schritt b bei dem Druck von 100 bis 350 bar, vorzugsweise 175 und 325 bar, besonders bevorzugt 200 bis 300 bar durchgeführt wird.

4. Verfahren nach einem der vorhergehenden Ansprüche, wobei der Anteil von 2,4,4-Trimethylpent-1-en im Di-isobutenstrom mindestens 60 Mol%, vorzugsweise mindestes 70 Mol% beträgt.

5. Verfahren nach einem der vorhergehenden Ansprüche, wobei die Abtrennung des homogenen Katalysatorsystems in Schritt c mittels thermischer Trennung und/oder Membrantrennung erfolgt.

6. Verfahren nach einem der vorhergehenden Ansprüche, wobei die Abtrennung des homogenen Katalysatorsystems in Schritt c mittels Verdampfung und anschließender Membrantrennung erfolgt.

7. Verfahren nach Anspruch 1 bis 5, wobei die Abtrennung des homogenen Katalysatorsystems in Schritt c mittels Membrantrennung erfolgt.

8. Verfahren nach Anspruch 7, wobei das homogene Katalysatorsystem im Retentat angereichert wird.

9. Verfahren nach einem der vorhergehenden Ansprüche, wobei der Di-isobutenstrom, der C4-bis C7-Olefinstrom und das homogene Katalysatorsystem, zunächst in einem Mischbehälter vermischt werden, bevor sie in die Reaktionszone geleitet werden.

10. Verfahren nach einem der vorhergehenden Ansprüche, wobei die destillative Aufarbeitung in Schritt d in einer einzigen Destillationskolonne erfolgt.

11. Verfahren nach Anspruch 10, wobei der Druck in der Destillationskolonne im Bereich von 0,3 bis 2 bar, bevorzugt im Bereich von 0,4 bis 1 bar besonders bevorzugt im Bereich von 0,5 bis 0,7 bar liegt.

12. Verfahren nach Anspruch 10 oder 11, wobei die Temperatur im Sumpf der Destillationskolonne in einem Bereich von 80 °C bis 160 °C liegt.

13. Verfahren nach einem der Ansprüche 10 bis 12, wobei die Temperatur am Kopf der Destillationskolonne in einem Bereich von 30 bis 80 °C liegt.

14. Verfahren nach einem der vorhergehenden Ansprüche, wobei in Schritt d die nicht umgesetzten Olefine abgetrennt und zur Hydroformylierung Schritt b zurückgeführt werden.

15. Verfahren nach einem der vorhergehenden Ansprüche, wobei ein C4-Olefin, vorzugsweise Isobuten, eingesetzt wird, wodurch als Aldehydgemisch eine Mischung aus 3,5,5-Trimethylhexanal und Pentanal, 2-Methylbutanal oder 3-Methylbutanal, vorzugsweise 3-Methylbutanal, erhalten wird.

## Claims

1. Process for hydroformylation of diisobutene and a C4 to C7 olefin, wherein the process comprises at least the following steps:
a. providing a diisobutene stream containing 2,4,4-trimethylpent-2-ene and 2,4,4-trimethylpent-1-ene and providing an olefin stream containing the C4 to C7 olefin;
b. hydroformylation of diisobutene and the C4 to C7 olefin with synthesis gas in the presence of a homogeneous catalyst system comprising at least Co or Rh and optionally a phosphorus-containing ligand in a reaction zone to obtain a preferably liquid product mixture comprising at least the aldehydes 3,5,5-trimethylhexanal and a C5 to C8 aldehyde formed by the hydroformylation, the homogeneous catalyst system and unreacted olefins;
c. removing the homogeneous catalyst system from the preferably liquid product mixture to obtain a crude product mixture comprising at least the aldehydes 3,5,5-trimethylhexanal and a C5 to C8 aldehyde formed by the hydroformylation and the unreacted olefins; and
d. distillative processing of the crude product mixture in at least one distillation column to remove the unreacted olefins to obtain an aldehyde mixture containing the aldehydes 3,5,5-trimethylhexanal and a C5 to C8 aldehyde formed.

2. Process according to Claim 1, wherein the hydroformylation in step b is performed at a temperature of 90°C to 250°C, preferably of 120°C to 200°C, particularly preferably of 120°C to 170°C.

3. Process according to Claim 1 or 2, wherein the hydroformylation in step b is performed at the pressure of 100 to 350 bar, preferably 175 to 325 bar, particularly preferably 200 to 300 bar.

4. Process according to any of the preceding claims, wherein the proportion of 2,4,4-trimethylpent-1-ene in the diisobutene stream is at least 60 mol%, preferably at least 70 mol%.

5. Process according to any of the preceding claims, wherein the removal of the homogeneous catalyst system in step c is effected by thermal separation and/or membrane separation.

6. Process according to any of the preceding claims, wherein the removal of the homogeneous catalyst system in step c is effected by evaporation and subsequent membrane separation.

7. Process according to Claim 1 to 5, wherein the removal of the homogeneous catalyst system in step c is effected by membrane separation.

8. Process according to Claim 7, wherein the homogeneous catalyst system accumulates in the retentate.

9. Process according to any of the preceding claims, wherein the diisobutene stream, the C4 to C7 olefin stream and the homogeneous catalyst system are initially mixed in a mixing vessel before they are passed into the reaction zone.

10. Process according to any of the preceding claims, wherein the distillative processing in step d is carried out in a single distillation column.

11. Process according to Claim 10, wherein the pressure in the distillation column is in the range from 0.3 to 2 bar, preferably in the range from 0.4 to 1 bar, particularly preferably in the range from 0.5 to 0.7 bar.

12. Process according to Claim 10 or 11, wherein the temperature in the bottom of the distillation column is in a range from 80°C to 160°C.

13. Process according to any of Claims 10 to 12, wherein the temperature at the top of the distillation column is in a range from 30°C to 80°C.

14. Process according to any of the preceding claims, wherein in step d the unreacted olefins are removed and recycled to hydroformylation step b.

15. Process according to any of the preceding claims, wherein a C4 olefin, preferably isobutene, is employed, as a result of which the aldehyde mixture obtained is a mixture of 3,5,5-trimethylhexanal and pentanal, 2-methylbutanal or 3-methylbutanal, preferably 3-methylbutanal.

## Revendications

1. Procédé d'hydroformylation de diisobutène et d'une oléfine en C4 à C7, ledit procédé comprenant au moins les étapes suivantes :
a. la fourniture d'un courant de diisobutène contenant du 2,4,4-triméthylpent-2-ène et du 2,4,4-triméthylpent-1-ène, et la fourniture d'un courant d'oléfine contenant l'oléfine en C4 à C7 ;
b. l'hydroformylation du diisobutène et de l'oléfine en C4 à C7 avec du gaz de synthèse en présence d'un système catalytique homogène comprenant au moins du Co ou du Rh et éventuellement un ligand contenant du phosphore, dans une zone de réaction, pour obtenir un mélange de produits de préférence liquide comprenant au moins les aldéhydes formés par l'hydroformylation 3,5,5-triméthylhexanal et un aldéhyde en C5 à C8, le système catalytique homogène et les oléfines n'ayant pas réagi ;
c. la séparation du système catalytique homogène du mélange de produits de préférence liquide pour obtenir un mélange de produits bruts comprenant au moins les aldéhydes formés par l'hydroformylation 3,5,5-triméthylhexanal et un aldéhyde en C5 à C8 et les oléfines n'ayant pas réagi ; et
d. le traitement par distillation du mélange de produits bruts dans au moins une colonne de distillation afin de séparer les oléfines n'ayant pas réagi pour obtenir un mélange d'aldéhydes contenant les aldéhydes formés 3,5,5-triméthylhexanal et un aldéhyde en C5 à C8.

2. Procédé selon la revendication 1, dans lequel l'hydroformylation à l'étape b est réalisée à une température de 90 à 250 °C, de préférence de 120 à 200 °C, de manière particulièrement préférée de 120 à 170 °C.

3. Procédé selon la revendication 1 ou 2, dans lequel l'hydroformylation à l'étape b est réalisée à une pression de 100 à 350 bar, de préférence de 175 à 325 bar, de manière particulièrement préférée de 200 à 300 bar.

4. Procédé selon l'une quelconque des revendications précédentes, dans lequel la proportion de 2,4,4-triméthylpent-1-ène dans le courant de diisobutène est d'au moins 60 % en moles, de préférence d'au moins 70 % en moles.

5. Procédé selon l'une quelconque des revendications précédentes, dans lequel la séparation du système catalytique homogène à l'étape c s'effectue au moyen d'une séparation thermique et/ou d'une séparation par membrane.

6. Procédé selon l'une quelconque des revendications précédentes, dans lequel la séparation du système catalytique homogène à l'étape c s'effectue au moyen d'une évaporation et d'une séparation par membrane ultérieure.

7. Procédé selon les revendications 1 à 5, dans lequel la séparation du système catalytique homogène à l'étape c s'effectue au moyen d'une séparation par membrane.

8. Procédé selon la revendication 7, dans lequel le système catalytique homogène s'accumule dans le rétentat.

9. Procédé selon l'une quelconque des revendications précédentes, dans lequel le courant de diisobutène, le courant d'oléfines en C4 à C7 et le système catalytique homogène sont d'abord mélangés dans un récipient de mélange avant d'être acheminés vers la zone de réaction.

10. Procédé selon l'une quelconque des revendications précédentes, dans lequel le traitement par distillation à l'étape d s'effectue dans une seule colonne de distillation.

11. Procédé selon la revendication 10, dans lequel la pression dans la colonne de distillation se situe dans la plage de 0,3 à 2 bar, de préférence dans la plage de 0,4 à 1 bar, de manière particulièrement préférée dans la plage de 0,5 à 0,7 bar.

12. Procédé selon la revendication 10 ou 11, dans lequel la température dans le fond de la colonne de distillation se situe dans une plage de 80 °C à 160 °C.

13. Procédé selon l'une quelconque des revendications 10 à 12, dans lequel la température à la tête de la colonne de distillation se situe dans une plage de 30 à 80 °C.

14. Procédé selon l'une quelconque des revendications précédentes, dans lequel, à l'étape d, les oléfines n'ayant pas réagi sont séparées et renvoyées à l'étape d'hydroformylation b.

15. Procédé selon l'une quelconque des revendications précédentes, dans lequel on utilise une oléfine en C4, de préférence l'isobutène, ce qui permet d'obtenir comme mélange d'aldéhydes un mélange de 3,5,5-triméthylhexanal et de pentanal, de 2-méthylbutanal ou de 3-méthylbutanal, de préférence de 3-méthylbutanal.
